# EUROPEAN PATENT SPECIFICATION

(11) **EP 1 313 523 B1**
(45) Date of publication and mention of the grant of the patent: **23.11.2005**
(21) Application number: 01962925.2
(22) Date of filing: 09.08.2001
(51) Int. Cl.: A61M 15/00

(54) **INHALATION DEVICE**
INHALATOR
DISPOSITIF D'INHALATION

(30) Priority: 29.08.2000 GB 0021024
(43) Date of publication of application: 28.05.2003
(73) Proprietor: GLAXO GROUP LIMITED, Greenford, Middlesex UB6 0NN (GB)
(72) Inventor: ROBERTSON, Duncan, Ware Herts. SG12 ODP (GB); ROBUSTI, George, Ware Herts. SG12 ODP (GB)
(74) Representative: Rice, Jason Neale
(86) International application number: PCT/EP2001/009202
(87) International publication number: WO 2002/017998

(56) References cited:
- US-A- 5 469 750
- US-A- 5 904 139

## Description

The present invention relates to an inhalation device for use in the administration of medicament to a patient.

### Background to the invention

The use of inhalation devices in the administration of medicaments, for example in bronchodilation therapy, is well known. Such devices generally comprise a body or housing within which a medicament container is located. A mouthpiece (or nozzle) is typically provided which communicates with the medicament container to allow passage of medicament from the source to the mouthpiece and thence, to the patient. When the patient breathes to take in the medicament, typically air is allowed to circulate throughout the body of the device to create airflow.

However, there are certain devices where it is advantageous that the volume of the body of the device that is exposed to airflow (and therefore moisture) is reduced. Typically such devices have moisture sensitive components such as medicament (for example dry powder) or electronic or other moisture sensitive equipment.

The applicants have now found that this problem can be solved by employing a device wherein the mouthpiece and the body of the device are separated such that the airflow between these parts is minimised. A specially created air hole in the mouthpiece and air channel in the body allow air to circulate through a part of the body not containing moisture sensitive components. These air holes and air channels are only able to communicate when the device is in a dispensing position. Preferably these air holes and channels are blocked during storage to prevent the entry of contaminants.

US-A-5 904 139 discloses an inhaler having a mouthpiece which is pivotally movable between dispensing and storage positions. US-A-5 469 750 discloses an inhaler having the features of the pre-characterising part of claim 1.

### Summary of the invention

According to the present invention there is provided an inhalation device for dispensing medicament as set forth in claim 1.

Preferably the inhalation device additionally comprises a monitor for monitoring the breath of a patient.

According to one embodiment of the invention said monitor comprises one or more sensors for sensing the pressure profile associated with the breath cycle.

According to another embodiment of the invention said monitor comprises one or more sensors for sensing the airflow profile associated with the breath cycle.

According to another embodiment of the invention said monitor comprises one or more sensors for sensing the temperature profile associated with the breath cycle.

According to a further embodiment of the invention said monitor comprises one or more sensors for sensing the moisture profile associated with the breath cycle.

According to a further embodiment of the invention said monitor comprises.one or more sensors for sensing the oxygen or carbon dioxide profile associated with the breath cycle.

Preferably said monitor sends an actuation signal to the actuator.

Preferably receipt of said actuation signal triggers release of medicament from a medicament carrier.

Preferably the collar portion additionally includes a microelectronic memory chip.

Preferably the body includes electrical contact pins. Preferably said electrical contact pins are connectable to a microelectronic system. Preferably said electronic contact pins are positioned such that they only contact the microelectronic memory chip when the actuator is in the dispensing position.

Preferably the device is provided with a dose counter.

Preferably the device is provided with a display window.

Preferably said aerosol container comprises a suspension of a medicament in a propellant.

Preferably said propellant comprises liquefied HFA134a, HFA-227 or carbon dioxide.

Preferably the medicament is selected from the group consisting of albuterol, salmeterol, fluticasone propionate and beclomethasone dipropionate and salts or solvates thereof and any mixtures thereof.

Preferably the medicament comprises salmeterol xinafoate and salts or solvates thereof and any mixtures thereof. More preferably the medicament comprises fluticasone propionate and salts or solvates thereof and any mixtures thereof. More preferably the medicament comprises a combination of salmeterol xinafoate and fluticasone propionate and salts or solvates thereof and any mixtures thereof.

Preferably at least a portion of the mouthpiece is shaped for ease of grip by the user.

The inhaler may be provided as a kit of parts.

Other features of the invention are set out in the sub-claims.

### Brief description of the drawings

The invention will now be described with reference to the accompanying drawings in which:
Figure 1 shows a perspective sectional view of an actuator and mouthpiece of an inhalation device according to the present invention.
Figure 2 shows a perspective view of a portion of a body according to the present invention.
Figures 3a and 3b show sectional views of the actuator and body of Figures 1 and 2 with the actuator and mouthpiece in the respective dispensing and storage positions.
Figure 4 shows a sectional view of an inhalation device herein with the mouthpiece in the storage position.
Figures 5a and 5b shows a perspective view of an aerosol container with a infra red sensor apparatus attached.

### Detailed description of the drawings

In the following description, those parts shown in the Figures which correspond to one another are assigned like numerals.

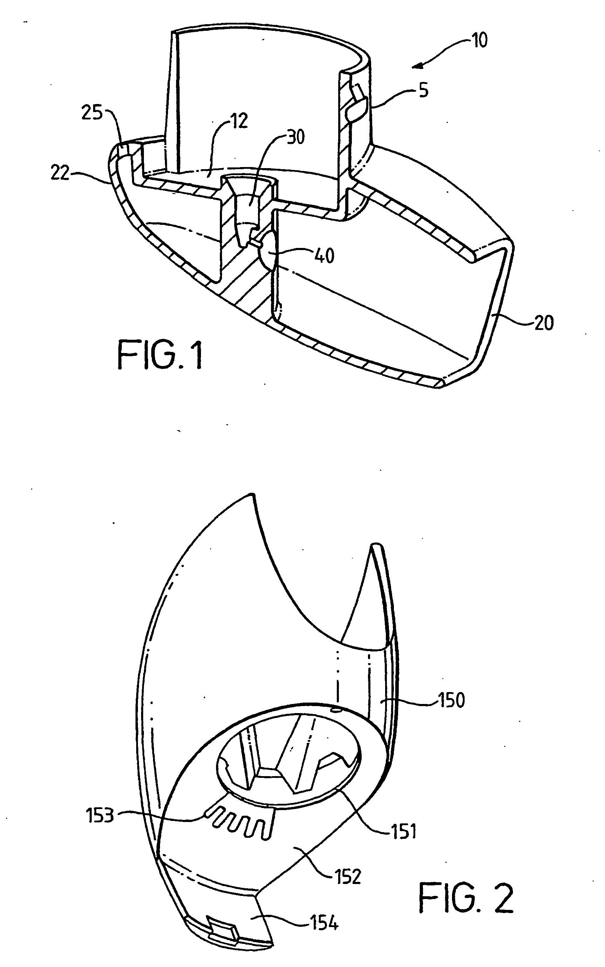

Figure 1 shows an actuator 10 for use in an inhalation device comprising, a collar portion 5; mouthpiece 20; stem block 30; and a passage defining a nozzle 40. The stem block 30 provides a support for the dispensing end of a valve stem of an aerosol container (not shown) and nozzle 40 allows for passage of the contents of the aerosol container into the mouthpiece for subsequent inhalation by the patient through the mouthpiece 20. The solid base portion 12 prevents air from entering the collar 10 and subsequently the body of the device (not shown), therefore preventing moisture from contacting any moisture sensitive apparatus located within the body. The mouthpiece 20 has air holes 25 located in the non-dispensing end 22. The air holes 25 communicate with air channels located in the body (not shown) to provide an air path.

Figure 2 shows a portion of the body 150. The body 150 has a hole 151 shaped to receive the collar of the actuator of figure 1 and a portion 152 shaped to fit with the mouthpiece of the actuator of figure 1. The actuator (not shown) may be inserted into the body 150 so that the collar is located in hole 151 and the mouthpiece lies against the portion 152 when it is in the storage position. End cap 154 covers the dispensing end of the mouthpiece, protecting it from ingress of contaminants. The body portion 152 contains a number of air channels 153 which are arranged in a finger pattern and pass through the surface of the body 150. The finger arrangement has been found to allow for a high level of air movement through the body 150, however the air channels 153 may be arranged in other formations, for example in a series of lines or grooves, or in another suitable pattern.

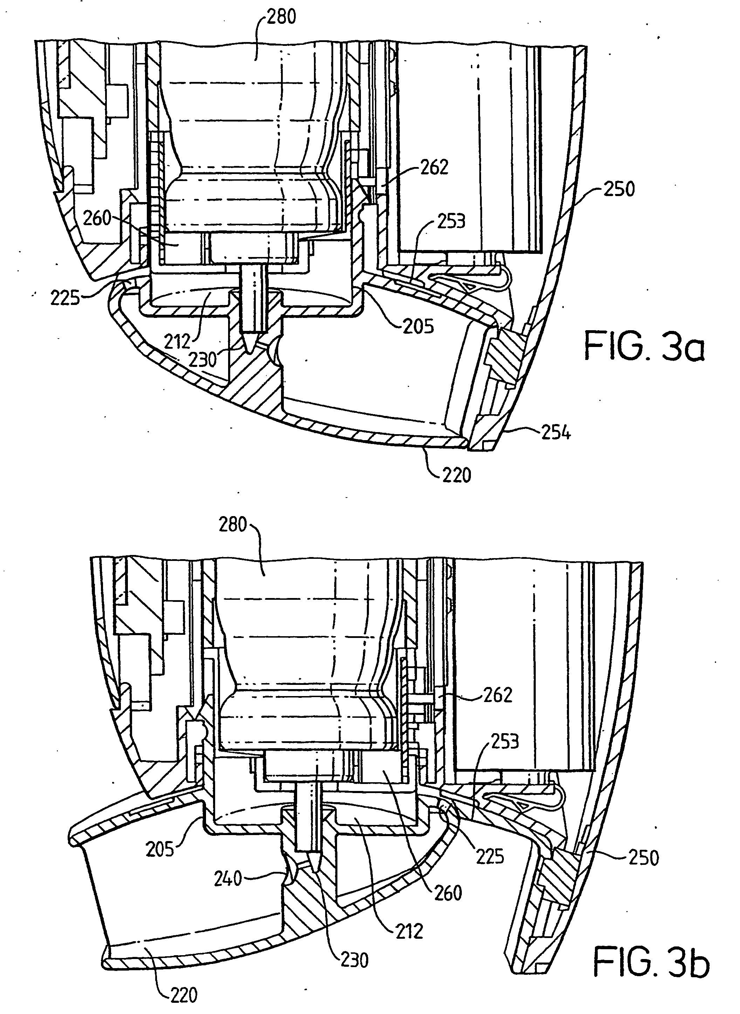

Figure 3a shows the actuator and body of Figures 1 and 2 in position with the mouthpiece 220 located in the storage position. The figure has been simplified to show only the actuator, a body 250 and aerosol container 280. The aerosol container 280 is inserted in the stem support 230, blocking airflow between the mouthpiece 220 and the collar portion 205. The solid base portion 212 of the collar portion 205 prevents airflow between the mouthpiece 220 and the rest of the inhalation device. The mouthpiece 220 is covered by end cap 254 of the body 250, preventing ingress of contaminants during storage. The air holes 225 in the mouthpiece 220 are blocked by the body 250 and the air channels 253 in the body 250 are blocked by the mouthpiece 220. There is therefore no communication between the body and the mouthpiece during storage. Also shown is microelectronic memory chip 260 located next to the aerosol container 280 and electrical contact pins 262 located within the body 250. In the storage position, the microelectronic memory chip 260 does not make electrical contact with the contact pins 262.

Figure 3b shows the device of Figure 3a with the mouthpiece located in the dispensing position. The aerosol container 280 is inserted in the stem support and will release medicament through the nozzle 240 into the mouthpiece 220 for inhalation by the patient. The solid base portion 212 of the collar portion 205 prevents air from entering the rest of the device when the patient breathes into the mouthpiece 220. The air hole 225 in the mouthpiece 220 is aligned with the air channels 253 in the body 250, allowing in-use circulation of air and creating airflow through the mouthpiece 220. The microelectronic memory chip 260 located next to the aerosol container 280 and the electrical contact pins 262 in the body are aligned when the mouthpiece is in the dispensing position such that electrical contact is made.

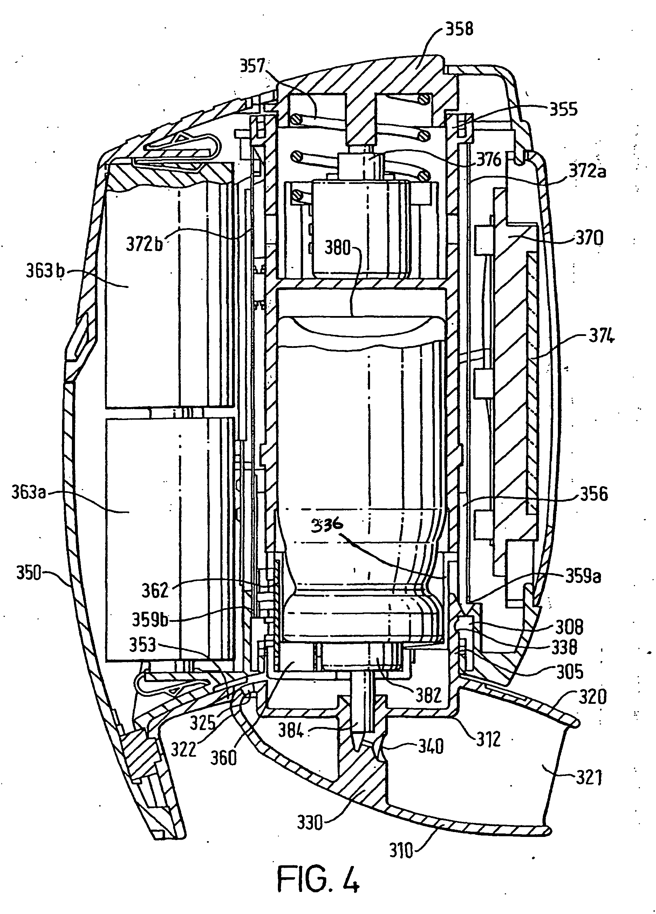

Figure 4 shows a metered dose inhaler for the delivery of medicament for inhalation by a patient. The inhaler comprises a housing body 350 in which an aerosol container 380 is located. An actuator in the form of a mouthpiece 320 is provided to the aerosol container 380.

The aerosol container 380 has a valve dispensing mechanism 382 in the form of a slide valve. Valve stem 384 connects with a stem support 330 which forms part of the actuator 310. The support 330 is provided with an outlet passage 340 enabling dispensed dose to pass through to the mouthpiece outlet 321. It will be appreciated that dispensing of the dose requires the aerosol container 380 to be depressed to actuate the slide valve dispensing mechanism 382 and dispense medicament into the outlet 321 from which it can be inhaled by a patient.

The actuator 310 has a tubular neck portion (collar) 305 for receipt of a collar 336 which itself engages the neck of the aerosol container 380. The tubular portion 305 is shaped such that collar 336 and so-engaged aerosol container are sufficiently movable in a sliding fashion therein to enable actuation of the slide valve mechanism 382. The aerosol container 380, collar 305 and actuator 310 together form a discrete unit which is reversibly removable from the body 350 when the actuator 310 is in certain orientations. The drawing shows the actuator in the 'endpiece secured orientation' in which retaining lip 308 makes snap-fit engagement with groove 338 provided in the tubular portion 305 of the actuator. A perpendicularly opposing point (i.e. 90° rotation therefrom) on the tubular portion 305 has no similar groove portion, such that when the actuator 310 is rotated through 90° to the 'endpiece unsecured orientation' the actuator 310, collar 305 and aerosol container may be removed from the housing body 350.

The mouthpiece 320 is separated from the collar 305 by solid base portion 312. The solid base portion 312 prevents air from entering the collar 305 and the interior of the device, therefore preventing moisture from contacting any moisture sensitive apparatus located therewithin. The mouthpiece 320 has air holes 325 located in the non-dispensing end 322. In the dispensing position, the air holes 325 communicate with air conduits 353 located in the body 350 to provide an air path. In the storage (i.e. 'end-piece unsecured') position, it will be appreciated that the air holes 325 do not communicate with the air conduits 353.

The collar 305 is provided with an electronic memory chip 360 which is capable of receiving data inputs and providing data outputs. The memory chip 360 connects via contact 362 to electronic circuitry (not visible) and power supply in the form of two lithium batteries 363a and 363b. The electronic circuitry further connects to electronic control system 370 which is capable of communication with the memory chip 360 and with various sensors on the device (for simplicity, not shown) and of providing visual output via display 374 to the patient.

It may be seen that the upper part of the aerosol container 380 is received by container seat 355. The container seat 355 is slidably movable within the housing along track 356 formed within the housing 350. The container seat 355 also comprises a spring actuator return 357 and actuator button 376 for use as a manual override. Plural lengths of shape memory alloy wire 372a, 372b (only two wires shown for simplicity) connect the container seat 355 to anchor positions 359a, 359b in the lower part of the housing. The wires 372a, 372b comprise a nickel-titanium alloy which contracts in response to the heating effect of the flow of electrical current therethrough. It may thus, be appreciated that when electrical current is passed through the plural lengths of wire 372a, 372b the container seat 355 will be drawn towards to the anchor positions 359a, 359b as the wires 372a, 372b contract. Actuation of the valve dispensing mechanism 382 and dispensing of medicament dose will thereby result. The flow of electrical current is controlled by the control system 370, which is itself responsive to inputs from various sensors (not shown) such as a sensor which senses the breath of a patient.

In the event of failure of electrical current flow it may be appreciated that a manual actuation button 358 may be manually pushed downwards to actuate the valve dispensing mechanism 382. The actuation step also results in the closing of switch 376 which records that a dose has been fired.

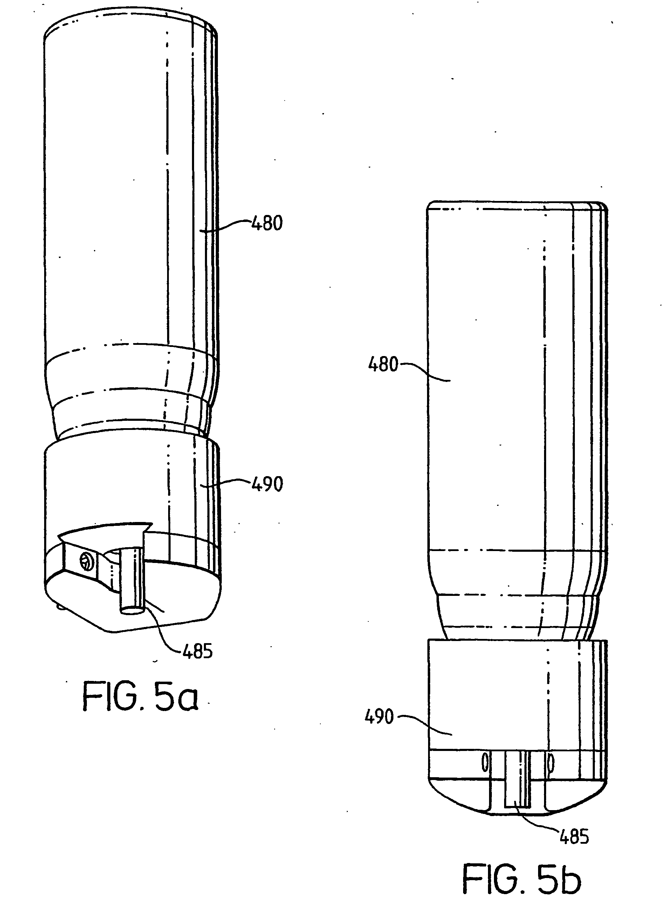

Figures 5a and 5b show aerosol container 480 with a collar 490 attached. The collar 490 is fixed such that the stem of the aerosol container 480 protrudes. The assembly of aerosol container 480 and collar 490 may be combined with an actuator similar to that shown in Figure 1 and a body similar to that shown in Figure 2 to make an assembly similar to that shown in Figures 3a and 3b.

The aerosol container and inhalation device of the invention is suitable for dispensing medicament, particularly for the treatment of respiratory disorders. Medicaments which may be administered in the aerosol formulations include any drug useful in inhalation therapy. Appropriate medicaments may thus be selected from, for example, analgesics, e.g., codeine, dihydromorphine, ergotamine, fentanyl or morphine; anginal preparations, e.g., diltiazem; antiallergics, e.g., cromoglycate (e.g. as the sodium salt), ketotifen or nedocromil (e.g. as the sodium salt); antiinfectives e.g., cephalosporins, penicillins, streptomycin, sulphonamides, tetracyclines and pentamidine; antihistamines, e.g., methapyrilene; anti- inflammatories, e.g., beclomethasone (e.g. as the dipropionate ester), fluticasone (e.g. as the propionate ester); flunisolide, budesonide, rofleponide, mometasone e.g. as the furoate ester), ciclesonide, triamcinolone (e.g. as the acetonide) or 6α, 9α-difluoro-11β-hydroxy-16α-methyl-3-oxo-17α-propionyloxy-androsta-1,4-diene-17β-carbothioic acid S-(2-oxo-tetrahydro-furan-3-yl) ester; antitussives, e.g., noscapine; bronchodilators, e.g., albuterol (e.g. as free base or sulphate), salmeterol (e.g. as xinafoate), ephedrine, adrenaline, fenoterol (e.g. as hydrobromide), formoterol (e.g. as fumarate), isoprenaline, metaproterenol, phenylephrine, phenylpropanolamine, pirbuterol (eg as acetate), reproterol (e.g. as hydrochloride), rimiterol, terbutaline (e.g. as sulphate), isoetharine, tulobuterol or 4-hydroxy-7-[2-[[2-[[3-(2-phenylethoxy)propyl]sulfonyl] ethyl]amino]ethyl-2(3H)-benzothiazolone; adenosine 2a agonists, eg 2R,3R,4S,5R)-2-[6-Amino-2-(1S-hydroxymethyl-2-phenyl-ethylamino)-purin-9-yl]-5-(2-ethyl-2H-tetrazol-5-yl)-tetrahydro-furan-3,4-diol (e.g. as maleate); α₄ integrin inhibitors eg (2S)-3-[4-({[4-(aminocarbonyl)-1-piperidinyl]carbonyl}oxy) phenyl]-2-[((2S)-4-methyl-2-{[2-(2-methylphenoxy)acetyl] amino}pentanoyl)amino] propanoic acid (e.g. as free acid or potassium salt), diuretics, e.g., amiloride; anticholinergics, e.g., ipratropium. (eg as bromide), tiotropium, atropine or oxitropium; hormones, e.g., cortisone, hydrocortisone or prednisolone; xanthines, e.g., aminophylline, choline theophyllinate, lysine theophyllinate or theophylline; therapeutic proteins and peptides, e.g., insulin or glucagon; vaccines, diagnostics, and gene therapies. It will be clear to a person skilled in the art that, where appropriate, the medicaments may be used in the form of salts, (e.g., as alkali metal or amine salts or as acid addition salts) or as esters (e.g., lower alkyl esters) or as solvates (e.g., hydrates) to optimise the activity and/or stability of the medicament.

Preferred medicaments are selected from albuterol, salmeterol, fluticasone propionate and beclomethasone dipropionate and salts or solvates thereof, e.g., the sulphate of albuterol and the xinafoate of salmeterol.

Medicaments can also be delivered in combinations. Preferred formulations containing combinations of active ingredients contain salbutamol (e.g., as the free base or the sulphate salt) or salmeterol (e.g., as the xinafoate salt) or formoterol (e.g. as the fumarate salt) in combination with an anti-inflammatory steroid such as a beclomethasone ester (e.g., the dipropionate) or a fluticasone ester (e.g., the propionate) or budesonide. A particularly preferred combination is a combination of fluticasone propionate and salmeterol, or a salt thereof (particularly the xinafoate salt). A further combination of particular interest is budesonide and formoterol (e.g. as the fumarate salt).

It will be understood that the present disclosure is for the purpose of illustration only and the invention extends to modifications, variations and improvements thereto within the scope of the appended claims.

## Claims

1. An inhalation device for dispensing medicament comprising:-
a body (150; 350), said body being shaped for receipt of an aerosol container (280;) which carries a medicament and has a valve stem (384); and
an actuator (10;) which is reversibly movable relative to the body between a
dispensing position and a storage position, said actuator having:-
a mouthpiece (20; 320);
a collar portion (5; 305) for receipt of the aerosol container, and
a stem block (30) for supporting the aerosol container, the stem block having a passageway which extends from an inlet opening, into which the valve stem is insertable, to an outlet opening, configured as a nozzle (40) providing communication between the mouthpiece and the aerosol container;
wherein said body includes at least one air channel (153;) and said actuator includes at least one air hole (25;) such that said air channel(s) communicates with said air hole(s) when the actuator is in said dispensing position and the air channel(s) and air hole(s) do not communicate when the actuator is in said storage position;
**characterised in that** the stem block extends through a solid base portion (12; 312) of the collar portion, which separates the collar portion from the mouthpiece, such that the inlet opening of the stem block is located on the collar portion side of the solid base portion and the outlet opening is located on the mouthpiece side of the solid base portion.

2. An inhalation device according to claim 1 wherein either the air channel or the at least one air hole is blocked when the actuator is in the storage position.

3. An inhalation device according to claim 1 wherein both the air channel and the at least one air hole are blocked when the actuator is in the storage position.

4. An inhalation device according to any of claims 1 to 3 wherein said air channel(s) forms a slot.

5. An inhalation device according to any of claims 1 to 4 wherein the body has a plurality of air channels.

6. An inhalation device according to claim 5 wherein said plurality of air channels are arranged in a finger arrangement.

7. An inhalation device according to claim 6 wherein said plurality of air channels are arranged in a series of grooves.

8. An inhalation device according to any of claims 1 to 7 wherein the at least one air hole is a slot.

9. An inhalation device according to any of claims 1 to 8 wherein the mouthpiece has a plurality of air holes.

10. An inhalation device according to any of claims 1 to 9 wherein the mouthpiece is covered by the body in the storage position and protrudes from the body in the dispensing position.

11. An inhalation device according to any of claims 1 to 10 wherein the mouthpiece is rotatably movable between the storage position and the dispensing position.

12. An inhalation device according to any of claims 1 to 11 wherein the collar portion additionally includes a microelectronic memory chip (360).

13. An inhalation device according to any of claims 1 to 12 wherein the body additionally includes electrical contact pins (362).

14. An inhalation device according to claim 13 wherein said electrical contact pins are connectable to a microelectronic system (370).

15. An inhalation device according to any of claims 12 to 14 wherein said electrical contact pins are positioned such that they only contact the microelectronic memory chip when the actuator is in the dispensing position and do not contact the microelectronic memory chip when the actuator is in the storage position.

16. An inhalation device according to any of claims 1 to 15 wherein the body is additionally provided with a sensor to detect the initial movement of the actuator from the storage position.

17. An inhalation device according to any of claims 1 to 16 additionally comprising the aerosol container.

18. An inhalation device according to claim 17 wherein the aerosol container and actuator are reversibly loadable into the body when the actuator is positioned midway between the storage and dispensing position.

19. An inhalation device according to claim 17 or 18 wherein said aerosol container comprises a suspension of a medicament in a propellant.

20. An inhalation device according to any of claims 1 to 19 wherein the body is shaped to be holdable in one hand and the actuator is movable from its storage position to its dispensing position by a motion of the thumb on the same hand.

## Patentansprüche

1. Inhalationsvorrichtung zur Abgabe eines Medikaments, mit:
einem Körper (150; 350), wobei der Körper für die Aufnahme eines Aerosolbehälters (280) geformt ist, welcher ein Medikament trägt und einen Ventilschaft (384) aufweist; und
einer Betätigungsvorrichtung (10), die reversibel relativ zu dem Körper, zwischen einer Abgabeposition und einer Lagerposition, bewegbar ist, wobei die Betätigungsvorrichtung aufweist:
ein Mundstück (20; 320);
einen Kragenabschnitt (5; 305) zur Aufnahme des Aerosolbehälters; und
einen Schaftblock (30) zum Stützen des Aerosolbehälters, wobei der Schaftblock einen Durchgangsweg aufweist, der sich von einer Einlassöffnung, in die der Ventilschaft einfügbar ist, zu einer Auslassöffnung erstreckt, die als eine Düse (40) konfiguriert ist, welche eine Verbindung zwischen dem Mundstück und dem Aerosolbehälter schafft;
wobei der Körper zumindest einen Luftkanal (153) aufweist, und die Betätigungsvorrichtung zumindest ein Luftloch (25) aufweist, derart, dass der/die Luftkanal/Luftkanäle mit dem/den Luftloch/Luftlöchern in Verbindung steht/stehen, wenn sich die Betätigungsvorrichtung in der Abgabeposition befindet, und der/die Luftkanal/Luftkanäle und das/die Luftloch/Luftlöcher nicht in Verbindung steht/stehen, wenn sich die Betätigungsvorrichtung in der Lagerposition befindet;
**dadurch gekennzeichnet, dass** sich der Schaftblock durch einen massiven Grundabschnitt (12; 312) des Kragenabschnitts, der den Kragenabschnitt von dem Mundstück trennt, derart erstreckt, dass sich die Einlassöffnung des Schaftblocks an der Kragenabschnitt-Seite des massiven Grundabschnitts befindet, und sich die Auslassöffnung an der Mundstückseite des massiven Grundabschnitts befindet.

2. Inhalationsvorrichtung nach Anspruch 1, bei der entweder der zumindest eine Luftkanal oder das zumindest eine Luftloch blockiert ist, wenn sich die Betätigungsvorrichtung in der Lagerposition befindet.

3. Inhalationsvorrichtung nach Anspruch 1, bei der beide, der zumindest eine Luftkanal und das zumindest eine Luftloch blockiert sind, wenn sich die Betätigungsvorrichtung in der Lagerposition befindet.

4. Inhalationsvorrichtung nach einem der Ansprüche 1 bis 3, bei welcher der/die Luftkanal/Luftkanäle einen Schlitz ausbildet/ausbilden.

5. Inhalationsvorrichtung nach einem der Ansprüche 1 bis 4, bei welcher der Körper eine Vielzahl von Luftkanälen aufweist.

6. Inhalationsvorrichtung nach Anspruch 5, bei der die Vielzahl von Luftkanälen in einer Finger-Anordnung angeordnet sind.

7. Inhalationsvorrichtung nach Anspruch 6, bei der die Vielzahl von Luftkanälen in einer Reihe von Nuten angeordnet sind.

8. Inhalationsvorrichtung nach einem der Ansprüche 1 bis 7, bei der das zumindest eine Luftloch ein Schlitz ist.

9. Inhalationsvorrichtung nach einem der Ansprüche 1 bis 8, bei der das Mundstück eine Vielzahl von Luftlöchern aufweist.

10. Inhalationsvorrichtung nach einem der Ansprüche 1 bis 9, bei der das Mundstück durch den Körper in der Lagerposition bedeckt wird, und von dem Körper in der Abgabeposition vorsteht.

11. Inhalationsvorrichtung nach einem der Ansprüche 1 bis 10, bei der das Mundstück zwischen der Lagerposition und der Abgabeposition drehbar bewegbar ist.

12. Inhalationsvorrichtung nach einem der Ansprüche 1 bis 11, bei welcher der Kragenabschnitt zusätzlich einen Mikroelektronik-Speicherchip (360) aufweist.

13. Inhalationsvorrichtung nach einem der Ansprüche 1 bis 12, bei welcher der Körper zusätzlich elektrische Kontaktstifte (362) aufweist.

14. Inhalationsvorrichtung nach Anspruch 13, bei der die elektrischen Kontaktstifte mit einem Mikroelektronik-System (370) verbindbar sind.

15. Inhalationsvorrichtung nach einem der Ansprüche 12 bis 14, bei der die elektrischen Kontaktstifte derart positioniert sind, dass sie den Mikroelektronik-Speicherchip lediglich berühren, wenn sich die Betätigungsvorrichtung in der Abgabeposition befindet, und den Mikroelektronik-Speicherchip nicht berühren, wenn sich die Betätigungsvorrichtung in der Lagerposition befindet.

16. Inhalationsvorrichtung nach einem der Ansprüche 1 bis 15, bei welcher der Körper zusätzlich mit einem Sensor versehen ist, um die Anfangsbewegung der Betätigungsvorrichtung aus der Lagerposition zu erfassen.

17. Inhalationsvorrichtung nach einem der Ansprüche 1 bis 16, zusätzlich mit einem Aerosolbehälter.

18. Inhalationsvorrichtung nach Anspruch 17, bei welcher der Aerosolbehälter und die Betätigungsvorrichtung reversibel in den Körper ladbar sind, wenn die Betätigungsvorrichtung in der Mitte zwischen der Lager- und Abgabeposition positioniert ist.

19. Inhalationsvorrichtung nach Anspruch 17 oder 18, bei welcher der Aerosolbehälter eine Suspension eines Medikaments in einem Treibmittel aufweist.

20. Inhalationsvorrichtung nach einem der Ansprüche 1 bis 19, bei welcher der Körper geformt ist, um in einer Hand haltbar zu sein, und die Betätigungsvorrichtung aus ihrer Lagerposition in ihre Abgabeposition durch eine Bewegung des Daumens der gleichen Hand bewegbar ist.

## Revendications

1. Dispositif d'inhalation pour la distribution de médicaments, comprenant :
un corps (150 ; 350), ledit corps étant configuré pour recevoir un récipient à aérosol (280;) qui contient un médicament et comporte une tige de valve (384) ; et
un dispositif d'actionnement (10 ;) qui peut être déplacé de façon réversible par rapport au corps entre une position de distribution et une position de rangement, ledit dispositif d'actionnement comprenant :
un embout (20 ; 320) ;
un élément de collerette (5 ; 305) pour recevoir le récipient à aérosol ; et
une embase de tige (30) pour supporter le récipient à aérosol, l'embase de tige comportant un passage qui s'étend depuis un orifice d'admission, dans lequel la tige de valve peut être insérée, jusqu'à un orifice de sortie, configuré en buse (40), assurant la communication entre l'embout et le récipient à aérosol ;
dans lequel ledit corps comprend au moins un passage d'air (153 ;) et ledit dispositif d'actionnement comprend au moins un évent (25 ;) de sorte que ledit ou lesdits passages d'air communiquent avec ledit ou lesdits évents lorsque le dispositif d'actionnement est dans ladite position de distribution et que le ou les passages d'air et évents ne communiquent pas lorsque le dispositif d'actionnement est dans ladite position de rangement ;
**caractérisé en ce que** l'embase de tige se prolonge à travers une base pleine (12 ; 312) de l'élément de collerette, ladite base séparant l'élément de collerette de l'embout, de sorte que l'orifice d'entrée de l'embase de tige est situé sur la face côté collerette de la base pleine et que l'orifice de sortie est situé sur la face côté embout de la base pleine.

2. Dispositif d'inhalation selon la revendication 1, dans lequel ledit au moins un passage d'air ou ledit au moins un évent est obturé lorsque le dispositif d'actionnement est dans la position de rangement.

3. Dispositif d'inhalation selon la revendication 1, dans lequel à la fois ledit au moins un passage d'air et ledit au moins un évent sont obturés lorsque le dispositif d'actionnement est dans la position de rangement.

4. Dispositif d'inhalation selon l'une quelconque des revendications 1 à 3, dans lequel ledit ou lesdits passages d'air forment une fente.

5. Dispositif d'inhalation selon l'une quelconque des revendications 1 à 4, dans lequel le corps comporte une pluralité de passages d'air.

6. Dispositif d'inhalation selon la revendication 5, dans lequel ladite pluralité de passages d'air sont disposés dans une configuration en forme de doigts.

7. Dispositif d'inhalation selon la revendication 6, dans lequel ladite pluralité de passages d'air sont disposés en une série de rainures.

8. Dispositif d'inhalation selon l'une quelconque des revendications 1 à 7, dans lequel ledit au moins un évent est une fente.

9. Dispositif d'inhalation selon l'une quelconque des revendications 1 à 8, dans lequel l'embout comporte une pluralité d'évents.

10. Dispositif d'inhalation selon l'une quelconque des revendications 1 à 9, dans lequel l'embout est recouvert par le corps dans la position de rangement et fait saillie par rapport au corps dans la position de distribution.

11. Dispositif d'inhalation selon l'une quelconque des revendications 1 à 10, dans lequel l'embout peut être déplacé en rotation entre la position de rangement et la position de distribution.

12. Dispositif d'inhalation selon l'une quelconque des revendications 1 à 11, dans lequel l'élément de collerette comprend en outre une puce mémoire microélectronique (360).

13. Dispositif d'inhalation selon l'une quelconque des revendications 1 à 12, dans lequel le corps comporte en outre des broches de contact électrique (362).

14. Dispositif d'inhalation selon la revendication 13, dans lequel lesdites broches de contact électrique sont connectables à un système à un système microélectronique (370) ;

15. Dispositif d'inhalation selon l'une quelconque des revendications 12 à 14, dans lequel lesdites broches de contact électrique sont positionnées de façon à n'être en contact avec la puce mémoire microélectronique que lorsque le dispositif d'actionnement est dans la position de distribution et à ne pas être en contact avec la puce mémoire microélectronique lorsque le dispositif d'actionnement est dans la position de rangement.

16. Dispositif d'inhalation selon l'une quelconque des revendications 1 à 15, dans lequel le corps est pourvu en outre d'un capteur pour détecter le début du mouvement du dispositif d'actionnement depuis la position de rangement.

17. Dispositif d'inhalation selon l'une quelconque des revendications 1 à 16, comprenant en outre le récipient à aérosol.

18. Dispositif d'inhalation selon la revendication 17, dans lequel le récipient à aérosol et le dispositif d'actionnement peuvent être chargés de façon réversible dans le corps lorsque le dispositif d'actionnement est positionné à mi-chemin entre la position de rangement et la position de distribution.

19. Dispositif d'inhalation selon la revendication 17 ou 18, dans lequel ledit récipient à aérosol contient une suspension d'un médicament dans un gaz propulseur.

20. Dispositif d'inhalation selon l'une quelconque des revendications 1 à 19, dans lequel le corps est configuré de façon à pouvoir être tenu dans une main et le dispositif d'actionnement est déplaçable de sa position de rangement vers sa position de distribution par un déplacement du pouce de la même main.
